# EUROPEAN PATENT SPECIFICATION

(11) **EP 0 224 252 B1**
(45) Date of publication and mention of the grant of the patent: **10.03.1993**
(21) Application number: 86116417.6
(22) Date of filing: 26.11.1986
(51) Int. Cl.: A61B 5/02, A61M 25/00, A61B 5/021

(54) **Catheter**
Katheter
Cathéter

(30) Priority: 27.11.1985 US 802296
(43) Date of publication of application: 03.06.1987
(73) Proprietor: Hewlett-Packard Company, Palo Alto, California 94304 (US)
(72) Inventor: Merrick, Edwin B., Stow MA.01775 (US)
(74) Representative: Liesegang, Roland, Dr.-Ing.

(56) References cited:
- DE-A- 3 130 367
- FR-A- 2 007 193
- US-A- 3 831 588
- IEEE TRANSACTIONS ON BIOMEDICAL ENGINEERING, vol. BME-20, no. 4, July 1973, pages 312-314, New York, US; W.D. FROBENIUS et al.: "A microminiature solid-state capacitive blood pressure transducer with improved sensitivity"
- IEEE TRANSACTIONS ON BIOMEDICAL ENGINEERING, vol. BME-20, no. 1, January 1973, pages 55-58, New York, US; R.W. MARTIN et al.: "Automated calibration of blood pressure signal conditioners"

## Description

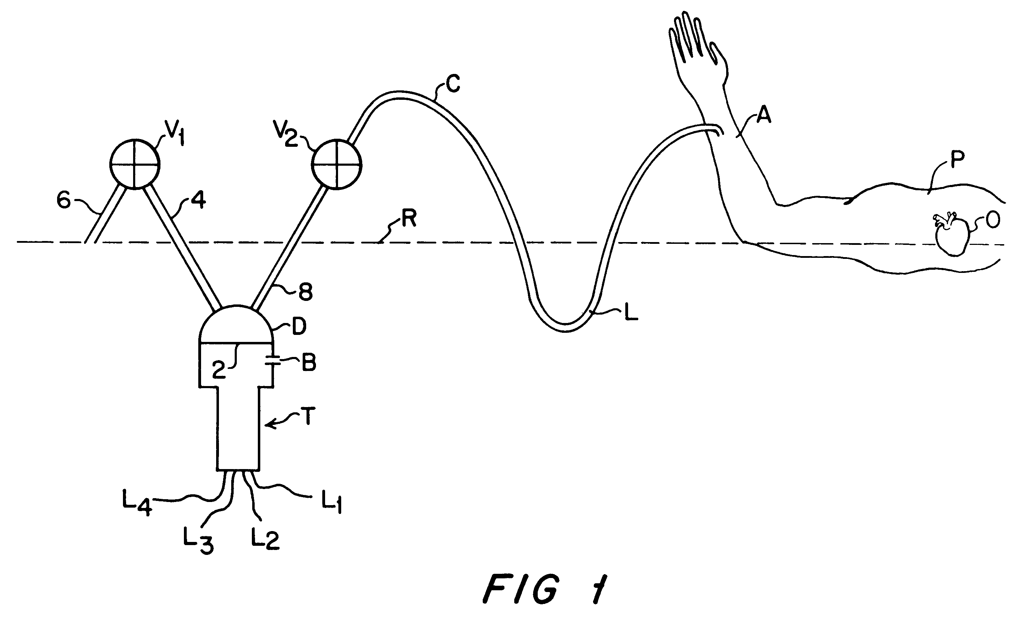

The most accurate way of measuring the blood pressure in the heart or other internal organ of a patient is to insert a catheter having a transducer at its distal end through a blood vessel to the point of interest, but this increases the risk of blood clots and may abrade tissue near the heart. Current practice avoids these problems by coupling blood pressure at a peripheral site, such as in the wrist, to a transducer outside of the patient's body with a saline-filled lumen in a catheter. In the illustration of this technique shown in FIGURE 1, an external transducer T is shown having a hollow pressure dome D that is mounted so as to apply the pressure in the dome D to a similar sensitive surface 2 that translates the pressure into a corresponding signal on output leads L₁ and L₂. Excitation is applied to the transducer T via leads L₃ and L₄. A tube 4 that communicates with the interior of the dome D is connected via a valve V₁ to a tube 6. Another tube 8 that communicates with the dome D is connected via a valve V₂ to a lumen in a catheter C, and the distal end of the catheter C is inserted into a blood vessel in the arm A of a patient P.

In use, the tubes 4, 6 and 8, the valves V₁ and V₂, the catheter C and the dome D are filled with a saline solution having nearly the same density as blood, and the catheter C is inserted into a blood vessel in the arm A of a patient p, e.g., in his wrist. The open end of the tube 6 is placed on a reference level indicated by the dashed line R that passes through the point at which the pressure is desired in the organ O. The valve V₂ is closed and the valve V₁ is opened. The signal on the leads L₁ and L₂ under this condition includes a first component due to the height of solution in the tube 6, in the tube 4 and in the valve V₁ above the sensing surface 2 and a second component called "transducer offset" that may add to or subtract from the first that is different for each transducer. The monitor or other device to which the leads L₁ and L₂ are coupled is then adjusted to a reading of zero pressure. Note that an opening B in the body of the transducer T permits atmospheric pressure to reach the underside of the sensing surface 2 so as to balance the effect of atmospheric pressure at the open end of the tube 6. Instead of manually adjusting the monitor to zero, the signal on L₁ and L₂ may be stored in a memory for numerical subtraction. Then the valve V₁ is closed and the valve V₂ opened so that the blood pressure of the patient at the organ O may be measured. A change in the height of the point in the arm A where the distal end of the catheter C is located has no effect on the pressure indicated by the transducer T.

A severe difficulty with such apparatus is that the compliance of the walls of the catheter C and the transducer T, as well as the inertia of the saline solution, impairs the frequency response so that the fidelity of the signal on the leads L₁ and L₂ is significantly less than optimum.

In order to improve accuracy, it has been proposed that the transducer be placed in a blood vessel in the body at a site remote from the organ generating the pressure of interest.

US-A 3 831 588 discloses a catheter-tip pressure sensor that produces an electric output proportional to pressure on it. The sensor is housed in a chamber in one end of the catheter. Means are provided for applying to the tube and to the interior of the pressure transmitting membran pressure changes of known magnitude. However, a new problem arises. Consider the case of a catheter inserted into the radial artery in the wrist of a human patient for the purpose of indicating the pressure in the aorta. In this situation, the transducer is advanced only a few inches beyond the patient's wrist toward the heart. If the patient elevates his arm such that the transducer is raised by 13.8 mm, the associated instrumentation will indicate a pressure decrease of 1 mm Hg. If one assumes that a patient has the ability to vary his wrist elevation by ±1.0 meter, then a pressure measurement error of ±72 mm Hg can result. One obvious solution to this problem is to strap the patient's wrist to his body at the level of the organ so as to prevent the patient from moving his arm. Even this extreme measure has its limitations since patients may roll or be rolled from side to side to prevent fluid pooling in the lungs and for other reasons. This orientation change would also change the elevation relationship between the organ and the transducer site and introduce an error.

Apparatus for measuring blood pressure in accordance with this invention is defined in claim 1. Preferably, the compliance means is a bubble of gas contained within the lumen so as to prevent deflection of the transducer diaphragm from exciting a fluid resonance between it, the fluid in the lumen, and the compliance of the lumen walls.

In use, the distal end of the catheter in which the transducer is mounted is inserted into a blood vessel in a limb of the patient, e.g., in his wrist. If the level of liquid in the reservoir is kept at the same level as the part in the body at which the blood pressure is being observed, the hydrostatic pressure applied via the lumen and compliance means to one side of the transducer is the same as the hydrostatic pressure applied to the other side by the blood, regardless of the position of the wrist, so that the only unbalanced variations in pressure applied to the transducer are those occurring at the point of interest. The purpose of the compliance means or fluid impedance means in the liquid path in the lumen is to prevent deflection of the transducer diaphragm from exciting resonance between the diaphragm, the liquid in the lumen and the compliance of the walls of the lumen.
FIGURE 1 illustrates a prior art system for measuring blood pressure;
FIGURE 2 illustrates a system incorporating the invention; and
FIGURE 2A is an enlarged view of the distal end of the catheter when one type of compliance means that might be used.

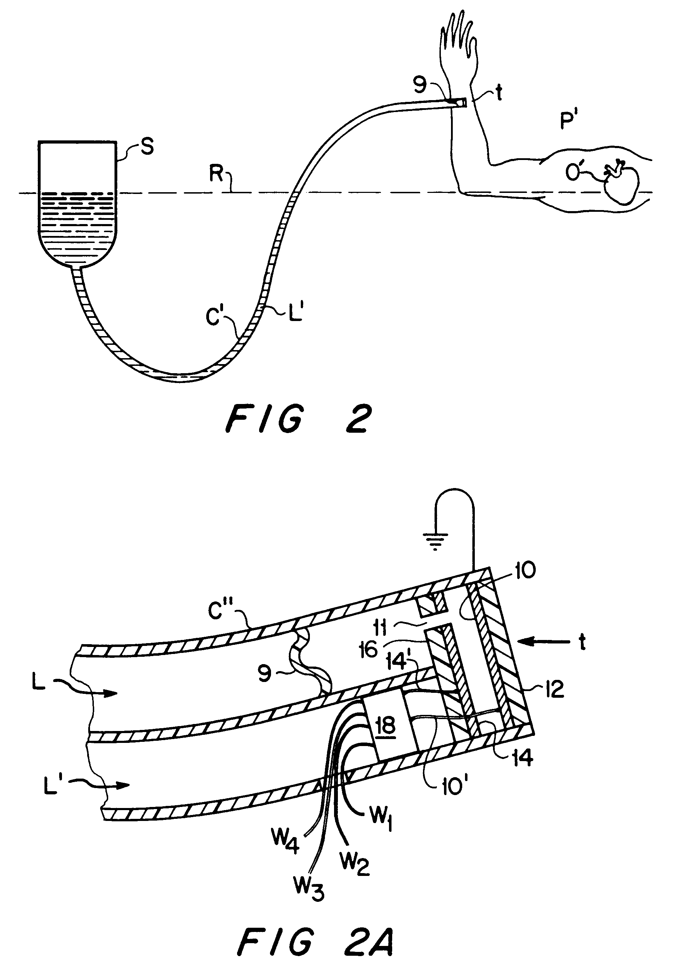

In FIGURE 2, components corresponding to FIGURE 1 are identified by the same letter primed. A reservoir S containing liquid that preferably has the same density as blood is shown as being connected to a transducer t via a catheter Cʹ having a lumen Lʹ therein. The lumen Lʹ is filled with the same liquid as the reservoir S, and the level of the liquid in the reservoir S is preferably placed at the reference level Rʹ which is at the same level as the point in the organ Oʹ of the body of a patient P at which the pressure is to be indicated. In this particular embodiment, a compliance means in the form of gas retained by a floppy membrane 9 is located in the liquid-filled path in the lumen L between the reservoir S and transducer t, and the space between the membrane 9 and the transducer t is filled with gas at ambient pressure. This prevents oscillating movement of the liquid in the lumen L as a result of interchange of energy via the liquid between the displacement of a diaphragm, not shown, in the transducer t and the compliance of the walls of the lumen L. Any suitable means can be used to energize the transducer t and derive information bearing signals from it.

FIGURE 2A is a detailed cross-sectional view of the distal end of the catheter Cʹ, the floppy membrane 9 and transducer t of an embodiment of the invention that is similar to that of FIGURE 2 except that the catheter Cʺ contains an additional lumen Lʹ through which wires carrying power to the transducer t and signals derived from it may be passed. The transducer t may take many forms but in FIGURE 2A it is shown as being comprised of a metal coating 10 on the inner surface of a flexible non-conductive plastic diaphragm 12 having an outer surface that is to be exposed to the blood pressure. A metal coating 14 is formed on a surface of an inflexible non-conducting bulkhead 16 and is separated from the metal coating 10 by a small space. A passageway 11 is provided through the bulkhead 16 and its metal coating 14. The space between the floppy membrane 9 and the metal coatings 10 and 14 is filled with gas at ambient pressure. The two metal coatings 10 and 14 form a capacitor whose capacitance is varied in accordance with the flexing of the diaphragm 12 by the blood pressure. An insulated lead 10ʹ that passes through the metal coating 14 and bulkhead 16 connects the metal coating 10 to a circuit contained in a block 18 within the lumen Lʹ, and an insulated lead 14ʹ that passes through the bulkhead 16 connects the coating 14 to the circuit in the block 18. The circuit functions to convert variations in the capacitance between the coatings 10 and 14 into an electrical signal which is placed on leads W₁ and W₂. Energization of the circuit is via leads W₃ and W₄. Whereas many circuits are known for performing this function, the one shown in my U.S. Patent No. 4,546,651 issued on October 15, 1985, and entitled "Transducer Coupling" would serve very well. The inside of the diaphragm 12 having the metal coating 10 is called the "reference side" of the diaphragm 12, and the other side is called the "active side". The active side is to be exposed to the blood pressure.

The gas pressure applied via the passageway 11 to the reference side of the diaphragm 12 is the same as any hydraulic pressure exerted by the liquid in the lumen Lʹ on the floppy membrane 9 and is also the same as the hydraulic pressure applied via the blood to the active side of the diaphragm 12. If a patient's arm is moved so as to raise the transducer t above the reference level Rʹ, both hydraulic pressures are reduced by the same amount; and if the arm moves so as to lower the transducer t below the reference level Rʹ, both hydraulic pressures increase by the same amount. In either case, the hydraulic pressures cancel one another so that the only unbalanced pressure applied to the diaphragm 12 is the blood pressure at the organ Oʹ.

In this particular embodiment of the invention, the compliance means is comprised of gas retained by the floppy membrane 9 sealed across the lumen L a short distance from the bulkhead 16. The space between it and the metal coatings 10 and 14 is filled with gas. Among the alternatives for the structure of the compliance means would be the inclusion of a soft closed cell foam elastomer in the lumen L, soft compliant walls in the lumen L itself, or a drop or two of oil such as silicone oil in the liquid in the lumen L so as to retain a gas bubble. With any suitable compliance means in the lumen L, there is little resonance between the compliance of the diaphragm 4, the mass of the liquid in the lumen L and the compliance of the walls of the lumen L occurs.

## Claims

1. Apparatus for deriving a signal corresponding to the blood pressure of a patient, comprising a
catheter (C',C''),
means defining a lumen (L,L') in said catheter, a transducer (t) mounted in one end of said lumen, said transducer (t) sealing the distal end of the catheter and having an active side and a reference side, the latter being toward said lumen, **characterised** by
a reservoir (S) for holding liquid, said reservoir being coupled to the other end of said lumen remote from said transducer, liquid contained in said lumen (L,L') and compliance means (9) in the liquid path between the reservoir (S) and the transducer (t) in said lumen.

2. Apparatus as in claim 1, **characterized** in that said compliance means comprises a floppy membrane (9) sealed across said lumen (L) and a fluid trapped between said membrane (9) and said transducer (t).

3. Apparatus as in claim 2, **characterized** in that said fluid is a gas or a liquid.

4. Apparatus as in claim 1 or 2, **characterized** in that said compliance means comprises oil within a portion of said lumen (L,L').

5. Apparatus as in claim 1, **characterized** in that the transducer (t) is formed by an indwelling pressure transducer (t), the active side of which faces said lumen (L,L') and said lumen apart from said compliance means contains a liquid.

## Patentansprüche

1. Vorrichtung zum Erzeugen eines Signales entsprechend dem Blutdruck eines Patienten mit einem Katheter (C', C"), Mitteln zum Bilden eines Volumens ("Lumen" L, L') in dem Katheter, einen am einen Ende des Volumens angeordneten Transducer (t), welcher das distale Ende des Katheters abdichtet und eine aktive Seite sowie eine Referenzseite aufweist, wobei letztere zu dem Volumen hinweist, **gekennzeichnet** durch ein Reservoir (S) zum Aufnehmen von Flüssigkeit, wobei das Reservoir an das andere Ende des Volumens entfernt von dem Transducer angeschlossen ist und Flüssigkeit in dem Volumen (L, L') enthalten ist, und durch eine Ausgleichsvorrichtung (9) in dem Flüssigkeitspfad zwischen dem Reservoir (S) und dem Transducer (t) in dem Volumen.

2. Vorrichtung nach Anspruch 1,
dadurch **gekennzeichnet,** daß die Ausgleichsvorrichtung eine flexible Membran (9) aufweist, welche das Volumen (L) abdichtet, und das Fluid zwischen der Membran (9) und dem Transducer (t) eingeschlossen ist.

3. Vorrichtung nach Anspruch 2,
dadurch **gekennzeichnet,** daß das Fluid ein Gas oder eine Flüssigkeit ist.

4. Vorrichtung nach Anspruch 1 oder 2,
dadurch **gekennzeichnet,** daß die Ausgleichsvorrichtung Öl in einem Abschnitt des Volumens (L, L') enthält.

5. Vorrichtung nach Anspruch 1,
dadurch **gekennzeichnet,** daß der Transducer (t) von einem eingefügten Druckaufnehmer (t) gebildet ist, dessen aktive Seite dem Volumen (L, L') gegenübersteht, und daß das Volumen außer der Ausgleichsvorrichtung eine Flüssigkeit enthält.

## Revendications

1. Dispositif destiné à évaluer un signal correspondant à la pression sanguine d'un patient, comportant
un cathéter (C',C"),
un dispositif définissant une lumière (L,L') dans ledit cathéter,
un transducteur (t) monté sur une extrémité de ladite lumière, ledit transducteur (t) fermant l'extrémité distale du cathéter et possédant un côté actif et un côté de référence, ce dernier se trouvant du côté de ladite lumière, caractérisé par
un réservoir (S) destiné à contenir un liquide, ledit réservoir étant couplé à l'autre extrémité de ladite lumière éloignée dudit transducteur,
un liquide contenu dans ladite lumière (L,L') et
un dispositif de conformité (9) sur le chemin du liquide situé entre le réservoir (S) et le transducteur (t) dans ladite lumière.

2. Dispositif selon la revendication 1, caractérisé en ce que ledit dispositif de conformité comporte une membrane souple (9) scellée dans ladite lumière (L) et un fluide piégé entre ladite membrane (9) et ledit transducteur (t).

3. Dispositif selon la revendication 1, caractérisé en ce que ledit fluide est un gaz ou un liquide.

4. Dispositif selon la revendication 1, caractérisé en ce que ledit dispositif de conformité comporte de l'huile à l'intérieur d'une partie de ladite lumière (L,L').

5. Dispositif selon la revendication 1, caractérisé en ce que ledit transducteur (t) est constitué d'un transducteur de pression situé dans un logement, son côté actif faisant face à ladite lumière (L,L') et ladite lumière séparée dudit dispositif de conformité contenant un liquide.
